# EUROPEAN PATENT SPECIFICATION

(11) **EP 3 065 822 B1**
(45) Date of publication and mention of the grant of the patent: **19.02.2020**
(21) Application number: 14809601.9
(22) Date of filing: 06.11.2014
(51) Int. Cl.: A61N 5/06

(54) **DEVICE FOR EMITTING LIGHT BY MEANS OF LEDS OF THE CONTINUOUS AND PULSED TYPE FOR AESTHETIC AND THERAPEUTIC TREATMENTS**
VORRICHTUNG ZUR LICHTEMISSION ANHAND VON KONTINUIERLICHEN UND GEPULSTEN LEDS FÜR ÄSTHETISCHE UND THERAPEUTISCHE BEHANDLUNGEN
DISPOSITIF EMETTANT DE LA LUMIERE AU MOYEN DE DEL DU TYPE CONTINU ET PULSE POUR DES TRAITEMENTS ESTHETIQUES ET THERAPEUTIQUES

(30) Priority: 07.11.2013 US 201314074149
(43) Date of publication of application: 14.09.2016
(73) Proprietor: Espansione Marketing S.p.A., 40050 Argelato, Frazione Funo (BO) (IT)
(72) Inventor: POMAR, Rodolfo, I-40050 Argelato, Frazione Funo (IT)
(74) Representative: Manzella & Associati
(86) International application number: PCT/EP2014/073974
(87) International publication number: WO 2015/067718

(56) References cited:
- WO-A1-2008/108774
- WO-A1-2011/112931
- WO-A1-2013/036558
- WO-A1-2014/146029
- WO-A2-2007/106856
- US-A1- 2006 217 690
- US-A1- 2010 076 529
- US-A1- 2012 150 044

## Description

### Technical Field

The present invention relates to a device for emitting light by means of LEDs of the continuous and pulsed type for aesthetic and therapeutic treatments of the skin and scalp.

### Background Art

It is known to resort to handpieces and apparatuses for the treatment of the skin and scalp which comprise irradiation sources (such as xenon lamps, halogen lamps, LEDs, discharge lamps, incandescent lamps and the like).

In many of these constructive solutions it is impossible to arrange in proximity the light source and the skin of the patient, since the light source generates large amounts of heat that might cause burns in said patient.

Constructive solutions that adopt cold light sources (or keep their temperature, even during operation, below threshold values, therefore at temperatures that are not dangerous for the patient) are however becoming increasingly established in relation to the reduction of the risk for the patient and therefore also to greater simplicity of use for the operator.

Furthermore, the aesthetic treatments that use LEDs as light sources are proving themselves particularly efficient for overcoming various skin problems (acne, impure skin, loss of tone and /or toning, wrinkles, dermatitis and others). Treatment devices comprising LEDs are described in the publication WO 2007/106856.

These treatments ensure the obtainment of optimum results when the distance of the light sources from the skin and the power of the beam emitted by the source are correlated according to formulas that are specific for each type of treatment apparatus.

Since the surface of the skin of the patient is not perfectly flat (it has instead different curvatures and physiological inflections, which are particularly evident in the face, in the hands, etc.), the power of the emitted beam is selected in relation to an average distance between the sources and the skin of the patient.

Therefore, some portions of the skin are subjected to the ideal treatment and other are overexposed (i.e., struck by a beam with higher than ideal power) because they are too close to the source and others will be underexposed (i.e., struck by a beam with lower than ideal power) because they are too far from the source.

For this reason, treatments that can be applied with apparatuses of the known type are generally scarcely efficient, offering poor results which in some cases do not allow to overcome the problem that is the subject of the treatment.

### Disclosure

The aim of the present invention is to solve the problems described above, proposing a device for emitting light by means of LEDs of the continuous and pulsed type for aesthetic and therapeutic treatments that allows to perform a uniform treatment of the entire skin surface that it faces.

Within this aim, an object of the invention is to propose a device for emitting light by means of LEDs of the continuous and pulsed type for aesthetic and therapeutic treatments that is particularly efficient and safe for the patient.

Another object of the invention is to propose a device for emitting light by means of LEDs of the continuous and pulsed type for aesthetic and therapeutic treatments that is simple to use for the operator.

A further object of the invention is to propose a device for emitting light by means of LEDs of the continuous and pulsed type for aesthetic and therapeutic treatments that has modest costs, is relatively simple to provide in practice and is safe in application.

This aim and these objects are achieved by a device for emitting light by means of LEDs of the continuous and pulsed type for aesthetic and therapeutic treatments as defined by the claims. The device comprises an electric power supply circuit which is connected to a respective external control and management unit, at least one LED controlled by said circuit and oriented toward a surface that is intended to face the skin of the patient in condition for use, the device has a platelike shape, said at least one LED is arranged on a laminar accommodation element that is arranged at a constant distance from the surface intended to face the skin of the patient in condition for use, said electric circuit comprises at least one apparatus for adjusting the electric power supply values of said at least one LED controlled by said control and management unit in order to set the power of the light beam emitted by said at least one LED.

There is also described an apparatus for aesthetic and therapeutic treatments comprising a control and management unit, at least one cable for connection to a treatment device provided with an electric power supply circuit for at least one LED that is oriented toward the surface of said device that is intended to face the skin of the patient in condition for use, said device is platelike and said at least one LED is arranged on a laminar accommodation element that is arranged at a constant distance from the surface that is intended to face the skin of the patient in conditions for use, said electric circuit comprising at least one apparatus for adjusting the electric power supply values of said at least one LED controlled by said control and management unit, in order to set the power of the light beam emitted by said at least one LED as a function of operating parameters contained within memory means of said unit.

Preferably, the at least one LED is arranged so that the distance of the light source from the skin of the patient in conditions for use is comprised in the range 0 - 5 cm.

### Description of Drawings

Further characteristics and advantages of the invention will become better apparent from the description of a preferred but not exclusive embodiment of the device for emitting light by means of LEDs of the continuous and pulsed type for aesthetic and therapeutic treatments, illustrated by way of nonlimiting example in the accompanying drawings, wherein:

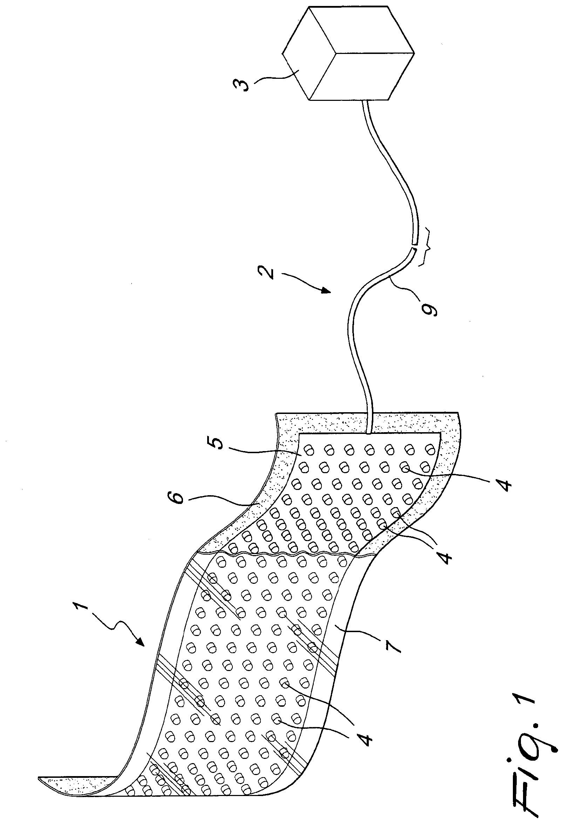

Figure 1 is a schematic perspective view of an embodiment of a device for emitting light by means of LEDs of the continuous and pulsed type for aesthetic and therapeutic treatments according to the invention;

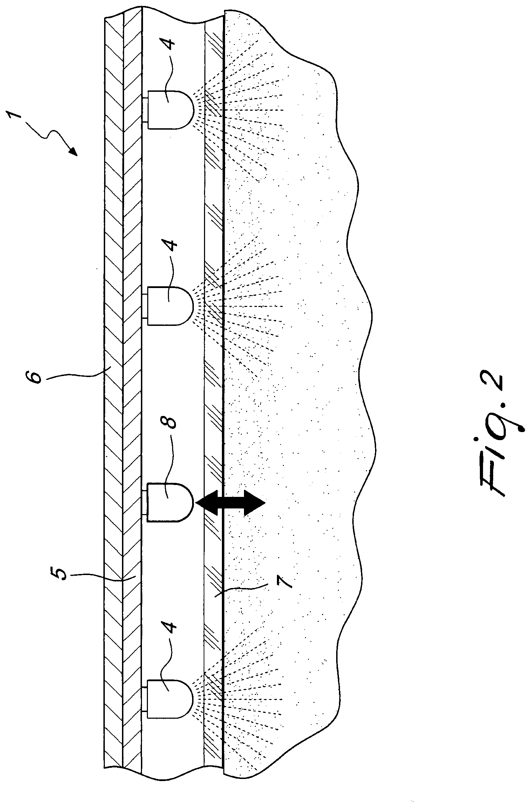

Figure 2 is a sectional view, taken along a transverse plane of a device for emitting light by means of LEDs of the continuous and pulsed type for aesthetic and therapeutic treatments according to the invention;

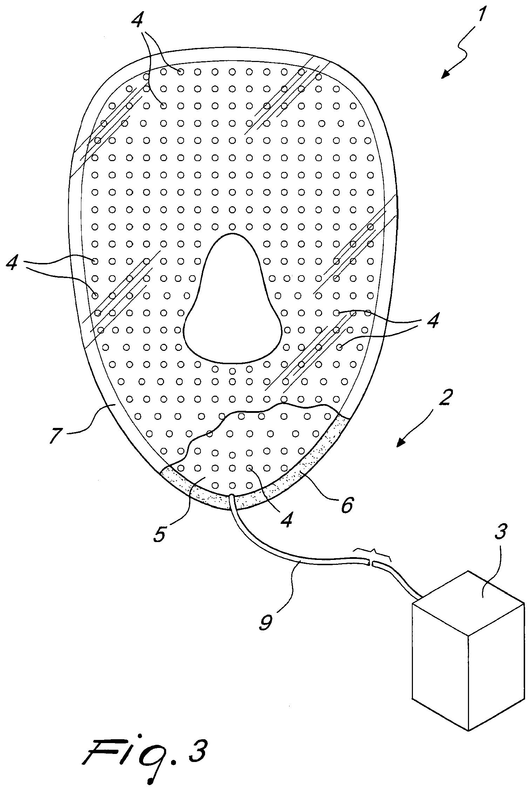

Figure 3 is a schematic perspective view of a further possible constructive solution of a device for emitting light by means of LEDs of the continuous and pulsed type for aesthetic and therapeutic treatments according to the invention;

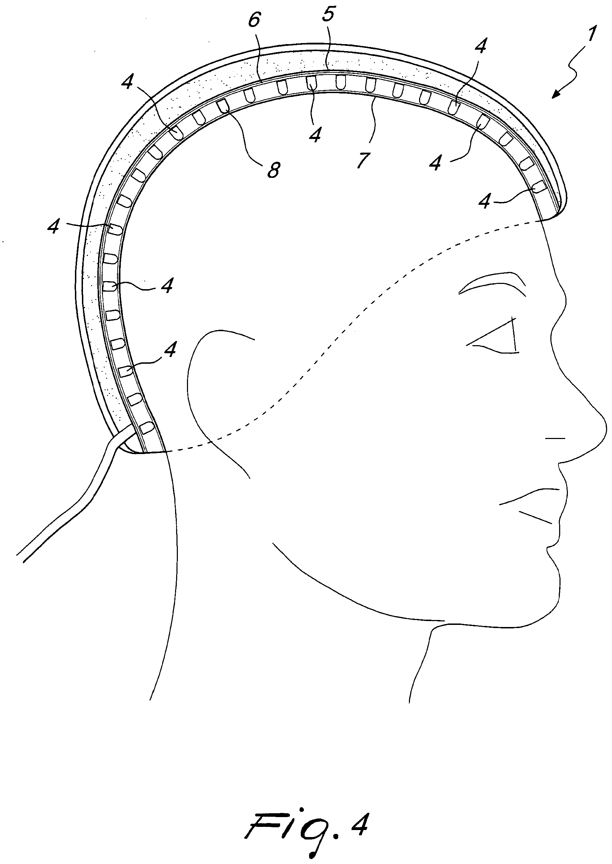

Figure 4 is a schematic perspective view of a further possible constructive solution of a device for emitting light by means of LEDs of the continuous and pulsed type for aesthetic and therapeutic treatments according to the invention.

### Best Mode

With particular reference to the above cited figures, the reference numeral 1 generally designates a device for emitting light by means of LEDs of the continuous and pulsed type for aesthetic and therapeutic treatments and the reference numeral 2 generally designates an apparatus for aesthetic treatments that comprises the device 1.

The device 1 comprises an electric power supply circuit connected to a respective external control and management unit 3 and at least one LED 4 controlled by the circuit and oriented toward a surface that is intended to face the skin of the patient on condition for use.

The device has a platelike shape and can be made of rigid or flexible material depending on the requirements of application.

The at least one LED 4 is arranged on a laminar accommodation element 5, which is arranged at a constant distance from the surface that is intended to face the skin of the patient in conditions for use.

The at least one LED (4) is arranged so that the distance of the light source from the skin of the patient in conditions for use is comprised in the range 0 - 5 cm.

The electric circuit comprises at least one apparatus for adjusting the electric power supply values of the at least one LED 4 controlled by the control and management unit 3 in order to set the power of the light beam emitted by the at least one LED 4.

In this manner, in other words, it is possible to adjust the power of the light beam emitted by the LED 4 (or by the plurality of LEDs 4) as a function of the specific requirements of application (i.e., as a function of the characteristics of the skin of the patient and of the skin dysfunction/pathology/irritation on which one intends to act).

According to a particular constructive solution of unquestionable practical interest, the electric circuit can comprise preferably a lamina for accommodating the respective components, comprising the at least one LED 4 and the at least one adjustment apparatus (in this case coinciding with the laminar element cited earlier).

The accommodation lamina is provided with surface conducting paths for the functional connection of the various components.

In particular, the lamina can be an ordinary printed circuit, a board for electronic circuits (normally known as PCB, printed circuit board), a flat support made of fibreglass and the like.

With reference to a particularly efficient application solution, the circuit can be interposed between an external panel 6 and a plate 7 that lies on the surface that is intended to face the skin of the patient in conditions for use.

The plate 7 is preferably made of material that is at least partially transparent to the radiation emitted by the at least one LED 4.

The external panel 6 instead is preferably made of opaque material so that the electric circuit and its components are not visible from the outside.

It should be pointed out conveniently that at least one component chosen among the lamina 5, the panel 6 and the plate 7 is made of flexible material.

This allows to arrange the device 1 on the skin of the patient so as to follow its physiological curves, thus ensuring that the correct distance of each LED 4 from the skin of said patient is maintained.

The device 1 comprises a sensor 8 that is intended to detect the light radiation reflected by the skin of the patient.

The control and management unit 3, as a function of the signal emitted by the sensor 8 following the detection of reflected light as a consequence of a test emission of light radiation by the at least one LED 4, sets the power of the light beam emitted by the at least one LED 4 (in the subsequent treatments) by means of the apparatus for adjusting the electric power supply values of the at least one LED 4.

In practice, it is therefore possible to emit a light pulse when the device 1 already faces the skin of the patient.

The sensor 8 (which can be of the type of a photodiode, a photocell, a photovoltaic cell and the like) detects a reflected light value, emitting a signal that is proportional thereto.

This signal indicates to the control and management unit 3 the skin type of the patient: very fair skins, dark skins, tanned skins, presence of irritations, etc.

As a function of the skin type of the patient (detected in this case by means of the sensor 8) and of the type of treatment that it is necessary to perform (in order to contrast acne, reduce wrinkles, cause reabsorption of an irritation), the unit 3 can then establish which is the suitable power of the beam that will be emitted by the LEDs 4.

If one adopts a device 1 without the sensor 8, the operator shall have to estimate the skin type of the patient in order to select the respective phototype and the corresponding characteristics on a control panel of the unit 3.

It is believed important to specify that the LEDs 4 are preferably a plurality, distributed on the laminar accommodation element 5 arranged at a constant distance from the surface intended to face the skin of the patient in conditions for use.

The distribution of the LEDs 4 can be uniform and equidistant or localized in predefined regions, in order to meet the specific requirements of application of each possible treatment.

The device 1 has a shape and dimensions that are complementary to those of a specific part of the human body for which it is intended.

The device is a mask that reproduces the shape of the face, a three-dimensional contour that reproduces the shape of the nose or of the cranium (for scalp treatments).

The control and management unit 3 comprises at least one cable 9 for connection to a treatment device 1 provided with an electric power supply circuit for at least one LED 4 that is oriented toward a surface of said device 1 that is intended to face the skin of the patient in conditions for use.

The device 1 has a platelike shape.

The at least one LED 4 is arranged on a laminar accommodation element 5 arranged at a constant distance from the surface that is intended to face the skin of the patient in conditions for use.

The electric circuit comprises at least one apparatus for adjusting the electric power supply values of the at least one LED 4 controlled by the control and management unit 3, in order to set the power of the light beam emitted by the at least on LED 4 as a function of operating parameters contained within memory means of the unit 3.

The unit 3 comprises the memory means intended to collect the treatment protocols related to all the skin problems for which the apparatus 2 is provided with the device 1 is intended.

Each protocol can be modified (in terms of power of the emitted beam and duration of the emission) as a function of the characteristics of the skin of the patient (phototype, inflammations in progress, specific pathologies).

It is specified that the control and management unit 3 can comprise for this purpose a selector of a plurality of different treatment protocols related to specific skin prolems and to specific skin types of the patient, said protocols being stored in said memory means.

The selector can be operated manually by the user as a function of the visual verification peformed by said user before proceeding with the treatment.

The device 1 comprises a sensor 8 intended to detect the light radiation reflected by the skin of the patient.

The control and management unit 3, as a function of the signal emitted by the sensor 8 following the detection of reflected light as a consequence of a test emission of light radiation by the at least one LED 4, is independently capable of setting the power of the light beam that will be emitted by the at least one LED 4 during the subsequent treatment by means of the apparatus for adjusting the electric power supply values of the at least one LED 4 as a function of the stored protocols.

Advantageously, the present invention solves the problems described earlier, proposing a device 1 for aesthetic treatments that allows to perform a uniform treatment of the entire skin surface that it faces.

This occurs by virtue of the specifically defined distance at which its LEDs are located with respect to the skin of the patient.

Effectively, the device 1 is very safe for the patient, since it allows to perform treatments that take into account the specific characteristics of the skin, avoiding overexposures (excessive power of the light beam that strikes the skin), which might cause damage, or under exposures (insufficient power of the light beam that strikes the skin), which would produce no beneficial effect.

Positively, the device 1 for aesthetic treatments is simple to use for the operator since it requires (in the case of non-automatic device 1) exclusively an estimate of the characteristics of the skin of the patient; in the case of a fully automatic device 1, no intervention on the part of the operator is required at all.

Validly, the device 1 can be manufactured at modest costs, since it is relatively simple to provide in practice and safe in application.

The invention is defined in the appended claims.

## Claims

1. Device (1) for emitting continuous and pulsed light by means of LEDs for aesthetic and therapeutic treatments, comprising an external control and management unit (3), an electric power supply circuit which is adapted to be connected to the external control and management unit (3), at least one LED (4) adapted to emit a light beam and controlled by said electric power supply circuit and oriented toward a surface that is intended to face the skin of the patient in conditions for use, wherein the device (1) has a platelike shape, its shape and dimensions being complementary to those of a specific part of the human body for which it is intended, said device being a mask that reproduces the shape of the face or a three-dimensional contour that reproduces the shape of the nose or of the cranium, said at least one LED (4) being arranged on a laminar accommodation element (5) that is arranged at a constant distance from the surface intended to face the skin of the patient in conditions for use, said at least one LED (4) being arranged so that the distance of the at least one LED from the skin of the patient in conditions for use is comprised in the range 0 - 5 cm, said electric power supply circuit comprising at least one apparatus for adjusting the electric power supply values of said at least one LED (4) configured to be controlled by said control and management unit (3) in order to set the power of the light beam emitted by said at least one LED (4) and wherein the device comprises a sensor (8) that is adapted to detect light radiation reflected by the skin of the patient and to emit a signal proportional to the detected light radiation, **characterised in that** said control and management unit (3) is configured to set the power of the light beam emitted by said at least one LED (4) through said apparatus for adjusting the electric power supply values of said at least one LED (4) as a function of the signal emitted by said sensor (8), following the detection of reflected light as a consequence of a test emission of light radiation by the at least one LED (4).

2. Device according to claim 1, wherein said electric circuit comprises the laminar accommodation element provided with conducting surface paths for the functional connection of components including said at least one LED (4) and said at least one apparatus for adjusting the electric power supply values of said at least one LED (4).

3. Device according to claim 1, wherein said electric power supply circuit is interposed between an external panel (6) and a plate (7) that lies on said surface intended to face the skin of the patient in conditions for use, said plate (7) being transparent to the radiation emitted by said at least one LED (4).

4. Device according to claim 3, wherein at least one component chosen among said laminar accommodation element (5), said external panel (6) and said plate (7) is made of flexible material.

5. Device according to claim 1, wherein said at least one LED (4) is a plurality of LEDs, distributed on said laminar accommodation element (5) that is arranged at a constant distance from the surface that is intended to face the skin of the patient in conditions for use.

6. Apparatus (2) for aesthetic and therapeutic treatments comprising a treatment device (1) being the device according to any one of the previous claims and at least one cable (9) for connection to said treatment device (1) wherein the control and management unit (3) comprises a memory means and is further configured to set the power level of the light beam emitted by said at least one LED (4) as a function of operating parameters contained within said memory means and as a function of protocols stored in said memory means.

## Patentansprüche

1. Vorrichtung (1) zum Emittieren von kontinuierlichem und gepulstem Licht mittels LEDs für ästhetische und therapeutische Behandlungen, die eine externe Steuer- und Verwaltungseinheit (3), eine elektrische Leistungsversorgungsschaltung, die angepasst ist, mit der externen Steuer- und Verwaltungseinheit (3) verbunden zu sein, mindestens eine LED (4), die angepasst ist, einen Lichtstrahl zu emittieren, und von der elektrischen Leistungsversorgungsschaltung gesteuert wird und auf eine Fläche ausgerichtet ist, die unter Gebrauchsbedingungen der Haut des Patienten zugewandt sein soll, umfasst, wobei die Vorrichtung (1) eine plattenartige Form aufweist, wobei ihre Form und Abmessungen jene eines spezifischen Teils des menschlichen Körpers, für den sie vorgesehen ist, ergänzen, wobei die Vorrichtung eine Maske ist, die die Form des Gesichts oder eine dreidimensionale Kontur reproduziert, die die Form der Nase oder des Schädels reproduziert, wobei die mindestens eine LED (4) auf einem laminaren Aufnahmeelement (5) angeordnet ist, das unter Gebrauchsbedingungen in einem konstanten Abstand von der Fläche angeordnet ist, die der Haut des Patienten zugewandt sein soll, wobei die mindestens eine LED (4) derart angeordnet ist, dass der Abstand der mindestens einen LED von der Haut des Patienten unter Gebrauchsbedingungen im Bereich von 0-5 cm umfasst ist, wobei die elektrische Leistungsversorgungsschaltung mindestens eine Einrichtung zum Anpassen der elektrischen Leistungsversorgungswerte der mindestens einen LED (4) umfasst, die dazu ausgelegt ist, von der Steuer- und Verwaltungseinheit (3) gesteuert zu werden, um die Leistung des Lichtstrahls, der von der mindestens einen LED (4) emittiert wird, einzustellen, und wobei die Vorrichtung einen Sensor (8) umfasst, der angepasst ist, eine Lichtstrahlung zu detektieren, die von der Haut des Patienten reflektiert wird, und ein Signal zu emittieren, das sich proportional zur detektierten Lichtstrahlung verhält, **dadurch gekennzeichnet, dass** die Steuer- und Verwaltungseinheit (3) dazu ausgelegt ist, die Leistung des Lichtstrahls, der von der mindestens einen LED (4) emittiert wird, über die Einrichtung zum Anpassen der elektrischen Leistungsversorgungswerte der mindestens einen LED (4) als eine Funktion des Signals, das vom Sensor (8) nach der Detektion des reflektierten Lichts als eine Konsequenz einer Testemission von Lichtstrahlung durch die mindestens eine LED (4) emittiert wird, einzustellen.

2. Vorrichtung nach Anspruch 1, wobei die elektrische Schaltung das laminare Aufnahmeelement umfasst, das mit leitenden Flächenpfaden für die Funktionsverbindung der Komponenten versehen ist, die die mindestens eine LED (4) und die mindestens eine Einrichtung zum Anpassen der elektrischen Leistungsversorgungswerte der mindestens einen LED (4) beinhalten.

3. Vorrichtung nach Anspruch 1, wobei die elektrische Leistungsversorgungsschaltung zwischen ein äußeres Paneel (6) und eine Platte (7), die auf der Fläche liegt, die der Haut des Patienten unter Gebrauchsbedingungen zugewandt sein soll, eingefügt ist, wobei die Platte (7) für die Strahlung, die von der mindestens einen LED (4) emittiert wird, transparent ist.

4. Vorrichtung nach Anspruch 3, wobei mindestens eine Komponente, die aus dem laminaren Aufnahmeelement (5), dem äußeren Paneel (6) und der Platte (7) ausgewählt ist, aus einem flexiblen Material besteht.

5. Vorrichtung nach Anspruch 1, wobei die mindestens eine LED (4) eine Vielzahl von LEDs ist, die auf dem laminaren Aufnahmeelement (5) verteilt sind, das in einem konstanten Abstand von der Fläche, die unter Gebrauchsbedingungen der Haut des Patienten zugewandt sein soll, angeordnet ist.

6. Einrichtung (2) für ästhetische und therapeutische Behandlungen, die eine Behandlungsvorrichtung (1), die die Vorrichtung nach einem der vorhergehenden Ansprüche ist, und mindestens ein Kabel (9) zum Verbinden der Behandlungsvorrichtung (1) umfasst, wobei die Steuer- und Verwaltungseinheit (3) ein Speichermittel umfasst und ferner dazu ausgelegt ist, den Leistungspegel des Lichtstrahls, der von der mindestens einen LED (4) emittiert wird, als eine Funktion von Betriebsparametern, die in dem Speichermittel enthalten sind, und als eine Funktion von Protokollen, die in dem Speichermittel gespeichert sind, einzustellen.

## Revendications

1. Dispositif (1) d'émission de lumière continue et pulsée au moyen de LED pour traitements esthétiques et thérapeutiques, comprenant une unité de contrôle et de gestion externe (3), un circuit d'alimentation électrique adapté pour être connecté à l'unité de contrôle et de gestion externe (3) au moins une LED (4) adaptée pour émettre un faisceau lumineux et contrôlée par ledit circuit d'alimentation électrique et orientée vers une surface destinée à être devant à la peau du patient dans des conditions d'utilisation, où le dispositif (1) a la forme d'une plaque, sa forme et ses dimensions étant complémentaires à celles d'une partie spécifique du corps humain pour laquelle il est destiné, ledit dispositif étant un masque reproduisant la forme du visage ou un contour en trois dimensions reproduisant la forme du nez ou du crâne, ladite au moins une LED (4) étant disposée sur un élément de logement laminaire (5) qui est disposé à une distance constante de la surface destinée à être devant à la peau du patient dans des conditions d'utilisation, ladite au moins une LED (4) étant disposée de sorte que la distance de la au moins une LED de la peau du patient dans des conditions d'utilisation soit comprise entre 0 et 5 cm, ledit circuit d'alimentation électrique comprenant au moins un appareil pour ajuster les valeurs d'alimentation électrique de ladite au moins une LED (4) configurée pour être contrôlée par ladite unité de contrôle et de gestion (3) afin de régler la puissance du faisceau lumineux émis par ladite au moins une LED (4) et où le dispositif comprend un capteur (8) adapté pour détecter un rayonnement lumineux réfléchi par la peau du patient et pour émettre un signal proportionnel au rayonnement lumineux détecté, **caractérisé en ce que** ladite unité de contrôle et de gestion (3) est configuré pour régler la puissance du faisceau lumineux émis par ladite au moins une LED (4) à travers ledit appareil pour régler les valeurs d'alimentation électrique de ladite au moins une LED (4) en fonction du signal émis par ledit capteur (8), suite à la détection de la lumière réfléchie résultant d'un test d'émission de rayonnement lumineux par la au moins une LED (4).

2. Dispositif selon la revendication 1, où ledit circuit électrique comprend l'élément de logement laminaire pourvu de chemins de surface conducteurs pour la connexion fonctionnelle de composants incluant ladite au moins une LED (4) et ledit au moins un appareil pour régler les valeurs d'alimentation électrique dudit au moins une LED (4).

3. Dispositif selon la revendication 1, où ledit circuit d'alimentation électrique est interposé entre un panneau externe (6) et une plaque (7) posée sur ladite surface destinée à être devant à la peau du patient dans des conditions d'utilisation, ladite plaque (7) étant transparent au rayonnement émis par ladite au moins une LED (4).

4. Dispositif selon la revendication 3, où au moins un composant choisi parmi ledit élément de logement laminaire (5), ledit panneau externe (6) et ladite plaque (7) est constitué d'un matériau flexible.

5. Dispositif selon la revendication 1, où ladite au moins une LED (4) est une pluralité de LED réparties sur ledit élément de logement laminaire (5) qui est disposé à une distance constante de la surface destinée à être devant à la peau du patient dans des conditions d'utilisation.

6. Appareil (2) pour des traitements esthétiques et thérapeutiques comprenant un dispositif de traitement (1), étant le dispositif selon l'une quelconque des revendications précédentes et au moins un câble (9) pour la connexion audit dispositif de traitement (1) où l'unité de contrôle et de gestion (3) comprend un moyen de mémoire et est en outre configurée pour régler le niveau de puissance du faisceau lumineux émis par ladite au moins une LED (4) en fonction des paramètres de fonctionnement contenus dans ledit moyen de mémoire, et en fonction des protocoles stockés dans ledit moyen de mémoire.
